# EUROPÄISCHE PATENTSCHRIFT

(11) **EP 0 579 827 B1**
(45) Veröffentlichungstag und Bekanntmachung des Hinweises auf die Patenterteilung: **18.04.2001**
(21) Anmeldenummer: 93914529.8
(22) Anmeldetag: 27.01.1993
(51) Int. Cl.: C07D 235/18, A61K 31/415

(54) **VERFAHREN ZUR HERSTELLUNG VON 2-ARYLBENZIMIDAZOL-5-SULFONSÄUREN**
METHOD OF PREPARING 2-ARYLBENZIMIDAZOLE 5-SULPHONIC ACIDS
PROCEDE DE FABRICATION D'ACIDES 2-ARYLBENZIMIDAZOL-5-SULFONIQUES

(30) Priorität: 04.02.1992 DE 4203072
(43) Veröffentlichungstag der Anmeldung: 26.01.1994
(73) Patentinhaber: MERCK PATENT GmbH, 64271 Darmstadt (DE)
(72) Erfinder: HEYWANG, Ulrich, D-6100 Darmstadt (DE); STEIN, Ingeborg, D-6106 Erzhausen (DE); FECHTEL, Ulrich, D-6105 Ober-Ramstadt (DE); CASUTT, Michael, D-6148 Heppenheim (DE); FALLER, Gerald, D-6140 Bensheim (DE); HÄRTNER, Hartmut, D-6109 Mühltal (DE)
(86) Internationale Anmeldenummer: EP9300179
(87) Internationale Veröffentlichungsnummer: WO9315061

(56) Entgegenhaltungen:
- FR-A- 1 450 505
- US-A- 3 655 632
- US-A- 4 309 551
- Chemical Abstracts, Band 84, Nr. 7, 14. August 1967, (Columbus, Ohio, US), K.K. PREOBRAZHENSKII et al.: "2-Alkylbenzimidazoles and their sulfo derivates", siehe Seite 454, Zusammenfassung Nr. 30963t, & ZH. PRIKL. KHIM. (LENINGRAD) 1975, 48(10), 2241-4
- Chemical Abstracts, Band 48, (Columbus, Ohio, US), L.S. EFROS: "Imidazole derivatives. VII. Preparation of sulfonic acids of benzimidazole by baking method", siehe Spalte 4524, & ZHUR. OBSHCHEI KHIM. 23, 835-41 (1953)

## Beschreibung

Die Erfindung betrifft ein verbessertes Verfahren zur Herstellung von 2-Arylbenzimidazol-5-sulfonsäuren der Formel I, 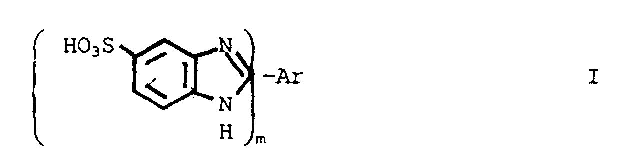 worin
- Ar: unsubstituiertes oder durch eine oder mehrere C₁-C₆ Alkyl- oder Alkoxygruppen substituiertes Phenyl und
- m: 1, 2 oder 3 bedeuten
wobei man o-Phenylendiamin in Gegenwart von Schwefelsäure mit einem Benzoesäurederivat der Formel II

Ar(-X)ₘ II

wobei Ar und m die angegebene Bedeutung besitzen, und X COO-Alkyl, worin Alkyl n-Alkyl mit 1 bis 6 C-Atomen bedeutet, COOH, COCl, COBr oder CN bedeutet, zwischen Raumtemperatur und 250 °C umsetzt. 2-Phenylbenzimidazol-5-sulfonsäure (Eusolex 232) ist ein wichtiger wasserlöslicher UV-B-Strahlenfilter mit einer großen Anwendungsbreite in der Kosmetik. Seine Verwendung als UV-Strahlenfilter wird z.B. beschrieben im Deutschen Reichspatent Nr. 676 103.

Seine Herstellung erfolgt z.B. nach V.G. Sayapin et al., KhGC [Chemistry of Heterocyclic Compounds] 6,1970,630-632 in einer Zweistufenreaktion, die von 1,2-Phenylendiamin und dem Bisulfitaddukt des Benzaldehyds bzw. von Phenylendiamin und Benzoesäure in Gegenwart von Polyphosphorsäure ausgehend 2-Phenylbenzimidazol hergestellt, welches dann mit Chlorsulfonsäure umgesetzt wird. Eine Übersicht über die Herstellung von 2-substituierten Benzimidazolen findet man z.B. in Chemical Reviews Vol. 74, No. 3, 1974 p 279 ff.

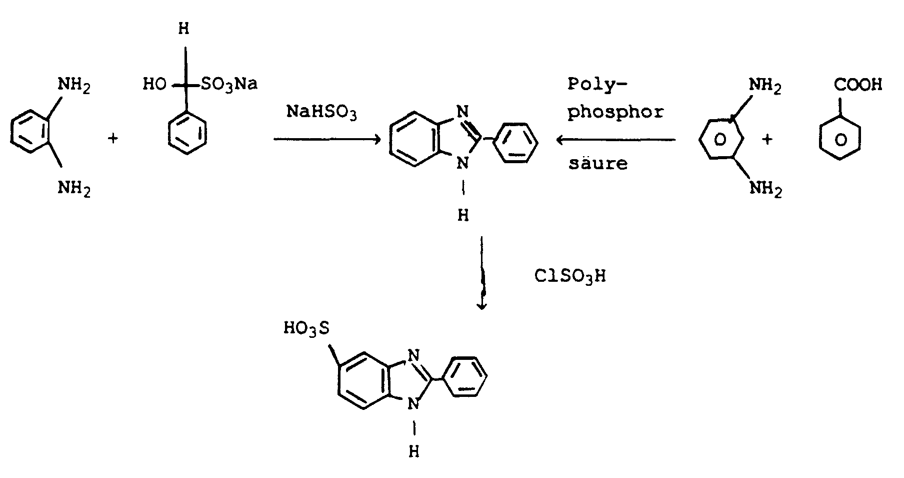

Dieses Verfahren hat jedoch große Nachteile:
a) Es ist ein Zweistufenverfahren und somit aufwendiger und teurer.
b) Der Umgang mit Chlorsulfonsäure in der Verfahrenstechnik ist problematisch, dabei können 2-Phenylbenzimidazoldisulfonsäuren entstehen, welche sich nur schlecht abtrennen lassen.
c) Bei den ersten Herstellungsverfahren von Phenylbenzimidazol muß Natriumhydrogensulfit im großen Überschuß eingesetzt werden, so daß im Verlauf der Aufarbeitung große Mengen Schwefeldioxid frei werden; dabei kann als Nebenprodukt 1-Benzyl-2-phenylbenzimidazol entstehen, welches sich nur schlecht abtrennen läßt. Bei dem zweiten Verfahren zur Herstellung von Phenylbenzimidazol aus Benzoesäure gelangt Phosphorsäure ins Abwasser, dies ist wegen der Gewässereutrophierung unerwünscht.

Die Nachteile für das oben erwähnte Verfahren lassen sich bequem umgehen: Es wurde überraschender Weise gefunden, daß bei der Kondensationsreaktion von 1,2-Phenylendiamin mit Benzoesäure in Schwefelsäure bei hohen Temperaturen Zwischenprodukte direkt sulfoniert werden, so daß ohne einer Zwischenisolierung direkt 2-Phenylbenzimidazol-5-sulfonsäure entsteht.

In der DE 12 82 855 wird die Verwendung von 1,3-Bis-(5-sulfobenzimidazol-2-yl)-benzol als wasserlöslicher UV-B-Strahlenfilter beschrieben.

Seine Herstellung erfolgt danach in Analogie zu 2-Phenylbenzimidazol in einem zweistufigen Verfahren.

Gegenstand der Erfindung ist somit ein verbessertes Verfahren zur Herstellung von 2-Arylbenzimidazol-5-sulfonsäuren der Formel I, 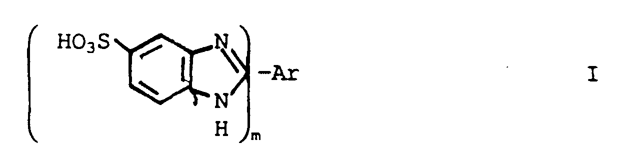 worin
- Ar: unsubstituiertes oder durch eine oder mehrere C₁-C₆ Alkyl- oder Alkoxygruppen substituiertes Phenyl, und bedeutet,
- m: 1, 2 oder 3 bedeuten,
wobei man o-Phenylendiamin in Gegenwart von Schwefelsäure mit einem Benzoesäurederivat der Formel II

Ar(-X)ₘ II

wobei Ar und m die angegebene Bedeutung besitzen und X COO-Alkyl, worin Alkyl n-Alkyl mit 1 bis 6 C-Atomen bedeutet, COOH, COCl, COBr oder CN bedeutet, zwischen Raumtemperatur und 250 °C umsetzt,
insbesondere wobei man 1 bis 10 mol, vorzugsweise 3 bis 8 mol, insbesondere 4 bis 7 mol Schwefelsäure bezogen auf 1 mol o-Phenylendiamin einsetzt.

Ein weiterer Gegenstand der Erfindung ist ein solches Verfahren, wobei man das Benzoesäurederivat in die Schwefelsäure einträgt, anschließend o-Phenylendiamin der Formel II hinzufügt, und auf 150 °C bis 220 °C erhitzt, oder wobei man o-Phenylendiamin und das Benzoesäurederivat gemeinsam in die Schwefelsäure einträgt.

Eine weitere bevorzugte Ausführungsform ist ein Verfahren wobei man die Schwefelsäure als Lösungsmittel einsetzt, insbesondere wobei man eine 50-100%ige Schwefelsäure einsetzt und/oder wobei das Lösungsmittel 1 bis 50 % Wasser enthält.

In einer besonders bevorzugten Ausführungsform des erfindungsgemäßen Verfahrens wird das Gemisch der Reaktanden etwa 1 bis 5 Stunden bei Temperaturen zwischen 80 und 140 °C gerührt, anschließend auf Temperaturen zwischen 170 und 250 °C erwärmt und weitere 0,5 bis 5 Stunden gerührt.

Die Durchführung des erfindungsgemäßen Verfahrens an sich ist einfach. Man legt die Schwefelsäure, vorzugsweise in Form einer 50-100%igen Lösung, insbesondere als konzentrierte ca. 96%ige Lösung vor. Dabei werden in der Regel 1 bis 10 mol, vorzugsweise 3 bis 8 mol, insbesondere 4 bis 7 mol Schwefelsäure bezogen auf 1 mol Phenylendiamin eingesetzt.

Der Zugabemodus der Reaktanden Benzoesäure und Phenylendiamin ist unkritisch. In der Regel gibt man einen der beiden Reaktanden bei Raumtemperatur hinzu, wobei sich das Gemisch auf in der Regel Temperaturen zwischen 80 und 140 °C erwärmt. Bei dieser Temperatur gibt man den 2. Reaktionspartner langsam hinzu, wobei sich die Temperatur der Reaktionsmischung gegebenenfalls weiter erhöht.

Anschließend wird das Reaktionsgemisch langsam auf Temperaturen zwischen 165 und 250 °C, vorzugsweise zwischen 175 und 200 °C, insbesondere zur Siedetemperatur des Gemisches, erwärmt und 1 bis 5 Stunden bei dieser Temperatur, gegebenenfalls unter Rühren, gehalten. Anschließend läßt man die Reaktionsmischung, vorzugsweise auf Temperaturen zwischen 100 und 150 °C abkühlen und fügt Wasser hinzu.

Nach kurzem Rühren, vorzugsweise 20 Minuten bis 2 Stunden, bei 50 bis 80 °C werden die festen Bestandteile abgetrennt, vorzugsweise mit warmen Wasser gewaschen und getrocknet.

Die Verbindungen der Formel I sind teilweise bekannt, teilweise neu. Die neuen Verbindungen der Formel I sind ebenfalls Gegenstand der Erfindung, insbesondere die Verbindungen der Formel Ia, wobei m 2 ist und Ar eine Gruppe der Formel 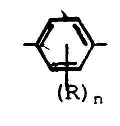 ist, worin R C₁₋₆-Alkyl oder Alkoxy und n 0, 1, 2, 3 oder 4 bedeutet. Überraschenderweise wurde gefunden, daß sich die Verbindungen der Formel Ia als wasserlöslicher UV-A-Filter eignen, wohingegen ähnliche, bekannte Verbindungen UV-B-Filter sind.

Zur Reinigung des getrockneten Rohproduktes wird dieses vorzugsweise in Wasser suspendiert und mit verdünnter Base neutralisiert. Nach Reinigung der so erhaltenen Lösung, vorzugsweise mit Aktivkohle, wird sie vorzugsweise auf Temperaturen zwischen 50 und 100 °C erwärmt und mit Mineralsäure, vorzugsweise konzentrierter Salzsäure sauer eingestellt. Die dabei ausfallende Sulfonsäure wird abgetrennt mit vorzugsweise warmen Wasser gewaschen und getrocknet.

In der Formel I bedeutet Ar unsubstituiertes oder durch ein oder mehrere C₁-C₆ Alkyl- oder Alkoxygruppen substituiertes Phenyl, vorzugsweise unsubstituiertes oder durch ein oder zwei, insbesondere in 4-, 3-, 3,4- oder 3,5-Position, C₁-C₃ Alkyl oder Alkoxygruppen substituiertes Phenyl, insbesondere unsubstituiertes Phenyl. Im Falle m = 2 bedeutet Ar vorzugweise 1, 3- oder 1,4-Phenylen insbesondere 1,4-Phenylen. Im Falle m = 3 bedeutet Ar vorzugsweise Benzol-1,3,5-triyl. In der Formel II bedeutet X COOAlkyl, worin Alkyl n-Alkyl mit 1 bis 6 C-Atomen, vorzugsweise 1 oder 2 C-Atomen bedeutet, COOH, COCl oder CN, vorzugsweise COOH, COOCH₃, COCl oder CN, ins- besondere COOH oder CN.

Das Verfahren hat gegenüber den obengenannten Verfahren folgende Vorteile:
a) Einfacher Einstufenprozeß, keine Schwierigkeiten mit pH-Wert-Kontrollen.
b) Das Produkt ist nicht mit 1-Benzyl-2-phenylbenzimidazol-6-sulfonsäure und 2-Phenylbenzimidazol-disulfonsäuren verunreinigt.
c) Es entsteht kein elementarer Schwefel, so daß das Abwasser einen wesentlich geringeren chemischen Sauerstoffbedarf hat.
d) Die Zwischenprodukte lassen sich ganz einfach und schnell abschleudern und waschen.
e) Die Ausbeute ist in vergleichbarer Größenordnung bzw. größer als bei dem aus dem Stand der Technik bekannten Verfahren.
f) Der Einsatz von Natriumhydrogensulfit erübrigt sich, so daß auch keine Schwefeldioxidhandhabung mehr von Nöten ist.

Weiterhin werden Kosmetische Zubereitungen bereitgestellt, die eine wirksame Menge mindestens einer Verbindung der Formel Ia in einem kosmetisch verträglichen Träger enthalten.

Vorzugsweise solche kosmetische Zubereitungen welche 0,1 bis 10 Gew.%, vorzugsweise 0,4 bis 1,0 Gew.%, insbesondere ca. 0,5 Gew.%, mindestens einer Verbindung der Formel Ia enthalten, insbesondere Zubereitungen welche zusätzlich einen UV-B-Filter enthalten.

Ein weiterer Gegenstand der Erfindung ist die Verwendung der Verbindungen der Formel Ia als kosmetisches Produkt, oder als Arzneimittel.

Weiterhin wird die Verbindung der Formel Ia zur Verwendung bei der vorbeugenden Behandlung von Entzündungen und Allergien der Haut, und zur Verwendung in der Prävention bestimmter Krebsarten bereitgestellt.

Pharmazeutische Zubereitungen, welche eine wirksame Menge mindestens einer Verbindung der Formel Ia in einem physiologisch unbedenklichen Träger oder Exzipienten enthalten, werden ebenfalls, insbesondere zur topischen Anwendung bereitgestellt.
Weiterhin wird die Verwendung der Verbindungen der Formel Ia als Arzneimittel bereitgestellt.
Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die nachfolgenden Beispiele sollen die Erfindung erläutern.

### Beispiel 1

In einem 1-l-Dreihalskolben werden 275 g (2,70 mol) 96%ige Schwefelsäure vorgelegt und anschließend 54,1 g (0,50 mol) 1,2-Phenylendiamin vorsichtig eingetragen. Zu der nunmehr ca. 120 °C warmen Suspension werden 85,5 g (0,70 mol) Benzoesäure gegeben und der Ansatz unter Rühren (klare Lösung ab etwa 130 °C) 5 Stunden auf ca. 200 °C erhitzt, wobei ein geringer Teil der Benzoesäure sublimiert. Danach läßt man auf etwa 150 °C abkühlen und rührt den Ansatz in 1 l kaltem Wasser ein, welches sich hierbei auf etwa 65 °C erwärmt. Man rührt noch eine Stunde bei 65 °C nach und saugt dann den Niederschlag ab und wäscht mit 80 °C warmen Wasser sulfatfrei. Das getrocknete Rohprodukt (83 g) wird in 830 ml Wasser suspendiert und der pH-Wert mit 32%iger Natronlauge auf 8 eingestellt. Nach Zugabe von 6,5 g Aktivkohle wird noch 30 Minuten bei Raumtemperatur gerührt und anschließend blankfiltriert. Das Filtrat wird auf 80 °C erwärmt und mit konzentrier Salzsäure ein pH-Wert von 3 eingestellt. Das ausgefallene Eusolex 232 wird warm abgesaugt, mit 2 l 90 °C warmen Wasser gewaschen und im Vakuumtrockenschrank bei 75 °C und 20 mbar zur Gewichtskonstanz getrocknet. Man erhält so 67,0 g (245 mmol, 49 % d.Th.) 2-Phenylbenzimidazol-5-sulfonsäure 410 °C (Zersetzung).

Analog werden hergestellt:
2-(4'-Methoxyphenyl)benzimidazol-5-sulfonsäure
2-(3'-Methoxyphenyl)benzimidazol-5-sulfonsäure
2-(4'-Ethoxyphenyl)benzimidazol-5-sulfonsäure
2-(3'-Ethoxyphenyl)benzimidazol-5-sulfonsäure
2-(3',5'-Dimethoxyphenyl)benzimidazol-5-sulfonsäure
2-(3',4'-Dimethoxyphenyl)benzimidazol-5-sulfonsäure
2-(3',5'-Diethoxyphenyl)benzimidazol-5-sulfonsäure
2-(3',4'-Diethoxyphenyl)benzimidazol-5-sulfonsäure

### Beispiel 2

Analog Beispiel 1 werden 72,2 g (0,70 mol) Benzonitril statt Benzoesäure eingesetzt. Man erhält 72,0 g (263 mmol, 53 % d.Th.) 2-Phenylbenzimidazol-5-sulfonsäure.

### Beispiel 3

Analog Beispiel 1 werden 95,3 g (0,70 mol) Benzoesäuremethylester statt Benzoesäure eingesetzt. Man erhält 64,0 g (233 mmol, 47 % d.Th.) 2-Phenylbenzimidazol-5-sulfonsäure.

### Beispiel 4

In einem 1-l-Dreihalskolben werden 275 g (2,70 mol) 96%ige Schwefelsäure vorgelegt und anschließend 85,5 g (0,70 mol) Benzoesäure eingetragen. Zu der Suspension werden vorsichtig 54,1 g (0,50 mol) 1,2-Phenylenamin zugegeben wobei der Ansatz sich auf etwa 100 °C erwärmt; dann wird der Ansatz unter Rühren (klare Lösung ab etwa 130 °C) 2 Stunden auf ca. 180 °C erhitzt, wobei ein geringer Teil der Benzoesäure sublimiert. Danach rührt man den Ansatz in 1 l kaltem Wasser ein, welches sich hierbei auf etwa 65 °C erwärmt. Man rührt noch eine Stunde bei 65 °C nach und saugt dann den Niederschlag ab und wäscht mit 80 °C warmen Wasser sulfatfrei. Das getrocknete Rohprodukt (83 g) wird in 830 ml Wasser suspendiert und der pH-Wert mit 32%iger Natronlauge auf 8 eingestellt. Nach Zugabe von 6,5 g Aktivkohle wird noch 30 Minuten bei Raumtemperatur gerührt und anschließend blankfiltriert. Das Filtrat wird auf 80 °C erwärmt und mit konzentrierter Salzsäure ein pH-Wert von 3 eingestellt. Das ausgefallene Eusolex 232 wird warm abgesaugt, mit 2 l 90 °C warmen Wasser gewaschen und im Vakuumtrockenschrank bei 75 °C und 20 mbar zur Gewichtskonstanz getrocknet. Man erhält so 67,0 g (245 mmol, 49 % d.Th.) 2-Phenylbenzimidazol-5-sulfonsäure.

### Beispiel 5

In einem 2-l-Vierhalskolben werden 182 ml destilliertes Wasser vorgelegt und dazu vorsichtig 1533 g (15 mol) 96%ige Schwefelsäure zugegeben. In die nunmehr etwa 100 °C warme Schwefelsäure werden unter kräftigem Rühren innerhalb von etwa 10 Minuten 367 g (3,0 mol) Benzoesäure eingetragen (die Temperatur fällt dabei auf etwa 80 °C ab). Anschließend wird auf 100 °C Innentemperatur angeheizt, dabei löst sich die Benzoesäure fast vollständig auf. Anschließend werden 325 g (3,0 mol) 1,2-Phenylendiamin so zudosiert, daß die Temperatur des Ansatzes 125 °C nicht übersteigt. Nach beendeter Zugabe wird der Ansatz auf Rückflußtemperatur (ca. 178 °C) erwärmt und weitere 6 Stunden bei dieser Temperatur gerührt, wobei ein geringer Teil der Benzoesäure sublimiert. Danach rührt man den Ansatz in 6 l 100 °C warmes Wasser ein, man rührt noch 30 Minuten bei 100 °C nach und saugt dann den Niederschlag ab und wäscht mit 2 l 80 °C warmen Wasser sulfatfrei. Das getrocknete Rohprodukt (580 g, 70 % d.Th.) wird in 2,5 l Wasser suspendiert und der pH-Wert mit 210 ml 32%iger Natronlaug auf 8 eingestellt. Nach Zugabe von 20 g Aktivkohle wird noch 60 Minuten bei Raumtemperatur gerührt und anschließend blankfiltriert. Das Filtrat wird auf 80 °C erwärmt und mit konzentrierter Schwefelsäure auf einen pH-Wert von 3 eingestellt. Das ausgefallene Produkt wird heiß abgesaugt, mit 4 l 90 °C warmen Wasser gewaschen und im Vakuumtrockenschrank bei 75 °C und 20 mbar zur Gewichtskonstanz getrocknet. Man erhält so 493 g (1,8 mol, 60 % d.Th.) 2-Phenylbenzimidazol-5-sulfonsäure.

### Beispiel 6

In einem 2 l-Vierhalskolben werden 1022 g (10 mol) 96%iger Schwefelsäure vorgelegt. Dazu werden unter kräftigem Rühren innerhalb von etwa 10 Minuten 166,1 g (1 mol) Terephthalsäure eingetragen. Eine 80 °C warme Lösung von 216,6 g (2,0 mol) 1,2-Phenylendiamin in 140 ml VE-Wasser wird so zur Schwefelsäure zudosiert, daß die Temperatur des Ansatzes 130 °C nicht übersteigt. Nach beendeter Zugabe wird der Ansatz auf 175 bis 180 °C erwärmt und etwa 4 Stunden bei dieser Temperatur gerührt. Während dieser Zeit destilliert man unter vermindertem Druck Wasser ab. Nach beendeter Reaktionszeit rührt man den heißen Ansatz in 4 l 80 °C warmes Wasser ein. Man läßt den Ansatz wieder auf 80 °C abkühlen, saugt den Niederschlag ab und wäscht mit 2 l 80 °C warmen Wasser. Das Rohprodukt wird in 2,5 l Wasser suspendiert und der pH-Wert mit 32%iger Natronlauge auf 8 eingestellt. Nach Zugabe von 20 g Aktivkohle wird noch 60 Minuten bei Raumtemperatur gerührt und anschließend blankfiltriert. Das Filtrat wird zunächst mit konz. Schwefelsäure auf einen pH von 6,0 eingestellt und dann auf 80 °C erwärmt. Anschließend wird mit konzentrierter Schwefelsäure auf einen pH-Wert von 2,0 eingestellt (Temperaturerhöhung auf etwa 100 °C). Man läßt den Ansatz wieder auf 80 °C abkühlen, saugt dann den Niederschlag ab und wäscht mit 1 l 80 °C warmen Wasser. (Test auf Sulfatfreiheit). Das Endprodukt wird im Vakuumtrockenschrank bei 75 °C und ca. 20 mbar zur Gewichtskonstanz getrocknet. Man erhält so 306 g (65 %) 1,4-Bis-(5-sulfobenzimidazol-2'-yl)-Benzol. Die Spektren der neuen Verbindungen entsprechen den Erwartungen (λₘₐₓ = 349 nm).

Analog werden hergestellt:
Aus Isophtalsäure:
   1,3-Bis-l(5-sulfobenzimidazol-2'-yl) -benzol (λₘₐₓ = 307 nm).
Aus 1,3,5-Benzoltricarbonsäure:
   1,3,5-Tris- (5-sulfobenzimidazol-2'-yl)-benzol

## Patentansprüche

1. Verfahren zur Herstellung von 2-Arylbenzimidazol-5-sulfonsäuren der Formel I, 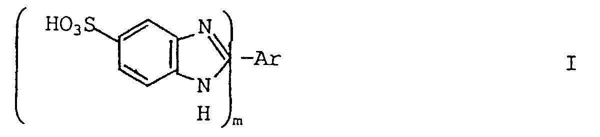 worin
Ar unsubstituiertes oder durch eine oder mehrere C₁-C₆ Alkyl- oder Alkoxygruppen substituiertes Phenyl, und
m 1, 2 oder 3 bedeuten,
dadurch gekennzeichnet, daß man o-Phenylendiamin in Gegenwart Schwefelsäure mit einem Benzoesäurederivat der Formel II
Ar-(X)ₘ II
wobei Ar die angegebene Bedeutung besitzt und X COO-Alkyl, worin Alkyl n-Alkyl mit 1 bis 6 C-Atomen bedeutet, COOH, COCl, COBr oder CN bedeutet, zwischen Raumtemperatur und 250 °C umsetzt.

2. Verfahren nach Anspruch 1, dadurch gekennzeichnet, daß man o-Phenylendiamin und das Benzoesäurederivat gemeinsam in die Schwefelsäure einträgt.

3. Verfahren nach einem der Ansprüche 1 bis 2, dadurch gekennzeichnet, daß man die Schwefelsäure als Lösungsmittel einsetzt.

4. Verfahren nach einem der Ansprüche 1 bis 3, dadurch gekennzeichnet, daß das Lösungsmittel 1 bis 50 % Wasser enthält.

5. Verfahren nach einem der Ansprüche 1 bis 4, dadurch gekennzeichnet, daß man das Gemisch der Reaktanden etwa 1 bis 5 Stunden bei Temperaturen zwischen 80 und 140 °C rührt anschließend auf Temperaturen zwischen 170 und 250 °C erwärmt und weitere 0,5 bis 5 Stunden rührt.

6. Verfahren nach einem der Ansprüche 1 bis 5, worin
m 1 bedeutet.

7. Verfahren nach einem der Ansprüche 1 bis 5, worin
m 2 bedeutet, und
Ar unsubstituiertes oder durch ein bis vier C₁-C₆-Alkyl- oder Alkoxygruppen substituierte 1,3- oder 1,4-Phenylengruppe bedeutet.

8. Verfahren nach einem der Ansprüche 1 bis 5, worin
m 3 bedeutet, und
Ar 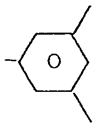 ist.

## Claims

1. Process for the preparation of 2-arylbenzimidazole-5-sulphonic acids of the formula I, 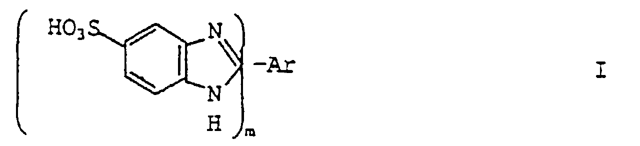 in which
Ar is phenyl, unsubstituted or substituted by one or more C₁-C₆-alkyl or alkoxy groups, and
m is 1, 2 or 3,
characterized in that o-phenylenediamine in the presence of sulphuric acid is reacted with a benzoic acid derivative of the formula II
Ar-(X)ₘ II
where Ar has the abovementioned meaning and X is COO-alkyl, in which alkyl is n-alkyl having 1 to 6 C atoms, COOH, COCl, COBr or CN, between room temperature and 250°C.

2. Process according to Claim 1, characterized in that o-phenylenediamine and the benzoic acid derivative are introduced jointly into the sulphuric acid.

3. Process according to one of Claims 1 to 2, characterized in that the sulphuric acid is used as solvent.

4. Process according to one of Claims 1 to 3, characterized in that the solvent contains 1 to 50% water.

5. Process according to one of Claims 1 to 4, characterized in that the mixture of the reactants is stirred for about 1 to 5 hours at temperatures between 80 and 140°C, is then heated to temperatures between 170 and 250°C and stirred for a further 0.5 to 5 hours.

6. Process according to one of Claims 1 to 5, in which
m is 1.

7. Process according to one of Claims 1 to 5, in which
m is 2, and
Ar is a 1,3- or 1,4-phenylene group, unsubstituted or substituted by one to four C₁-C₆-alkyl or alkoxy groups.

8. Process according to one of Claim 1 to 5, in which
m is 3, and
Ar is

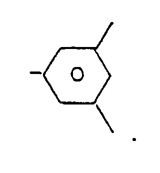

## Revendications

1. Procédé de préparation d'acides 2-arylbenzimidazol-5-sulfoniques de formule I, 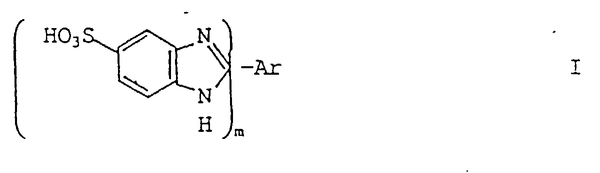 dans laquelle
Ar représente un phényle non substitué ou substitué par un ou plusieurs groupes alkyle ou alcoxy en C₁ à C₆, et m vaut 1, 2 ou 3,
caractérisé en ce qu'on fait réagir une o-phénylène diamine en présence d'acide sulfurique avec un dérivé d'acide benzoïque de formule II
Ar-(X)ₘ II
dans laquelle Ar a la signification indiquée et X représente un COO-alkyle, dans lequel alkyle est un n-alkyle comportant de 1 à 6 atomes de carbone, COOH, COCl, COBr ou CN, entre la température ambiante et 250°C.

2. Procédé selon la revendication 1, caractérisé en ce qu'on introduit la o-phénylène diamine et le dérivé d'acide benzoïque ensemble dans l'acide sulfurique.

3. Procédé selon l'une des revendications 1 à 2, caractérisé en ce qu'on utilise l'acide sulfurique comme solvant.

4. Procédé selon l'une des revendications 1 à 3, caractérisé en ce que le solvant contient de 1 à 50 % d'eau.

5. Procédé selon l'une des revendications 1 à 4, caractérisé en ce qu'on agite le mélange des réactifs pendant environ 1 à 5 heures à des températures comprises entre 80 et 140°C, puis qu'on le chauffe à des températures comprises entre 170 et 250°C et qu'on l'agite encore pendant 0,5 à 5 heures.

6. Procédé selon l'une des revendications 1 à 5, dans lequel
m vaut 1.

7. Procédé selon l'une des revendications 1 à 5, dans lequel
m vaut 2, et
Ar représente groupe 1,3- ou 1,4-phénylène non substitué ou substitué par de 1 à 4 groupes alkyle ou alcoxy en C₁ à C₆.

8. Procédé selon l'une des revendications 1 à 5, dans lequel
m vaut 3 et
Ar représente
